# EUROPEAN PATENT SPECIFICATION

(11) **EP 2 768 095 B1**
(45) Date of publication and mention of the grant of the patent: **19.09.2018**
(21) Application number: 11873806.1
(22) Date of filing: 14.10.2011
(51) Int. Cl.: H01T 19/00, H01T 23/00, A61L 9/22

(54) **APPARATUS FOR GENERATING ELECTRIC FIELD AND ELECTRIC DISCHARGE**
VORRICHTUNG ZUR ERZEUGUNG EINES ELEKTRISCHEN FELDES UND EINER ELEKTRISCHEN ENTLADUNG
CHAMP ÉLECTRIQUE, ET DISPOSITIF DE GÉNÉRATION DE DÉCHARGE

(43) Date of publication of application: 20.08.2014
(73) Proprietor: Mitsubishi Electric Corporation, Tokyo 100-8310 (JP)
(72) Inventor: FURUHASHI, Takuya, Tokyo 100-8310 (JP); MORIOKA, Reiji, Tokyo 100-8310 (JP)
(74) Representative: Pfenning, Meinig & Partner mbB
(86) International application number: PCT/JP2011/073602
(87) International publication number: WO 2013/054429

(56) References cited:
- JP-A- 2005 074 389
- JP-A- 2005 205 405
- JP-A- 2005 205 405
- JP-A- 2007 007 589
- JP-A- 2007 007 589
- JP-B2- 4 252 405
- US-A- 4 713 092

## Description

### Technical Field

The present invention relates to an electric field and discharge generator that forms an electric field to generate discharge between electrodes.

### Background Art

US 4,713,092 A discloses: An electrostatic precipitator includes charging, inertia and collection sections, which are arranged in the order mentioned along the direction of gas flow containing therein particles to be removed. The charging section includes a parallel electrode and a discharging electrode located between the parallel plates, and, thus, the particles floating in the gas passing through the charging section become charged and partly agglomerated. The inertia section includes a deflector plate arranged at a predetermined angle thereby causing the gas flow to change its course of action locally to have the particles impinge on the deflector plate due to inertia. The collection section includes a trough which is formed continuous with the deflector plate with its mouth opened against the gas flow.

Patent Literatures 1 and 2 described below disclose electric dust collectors of related art.

The dust collector described in Patent Literature 1 includes an ionizing section that ionizes sucked dust particles. The ionizing section includes a high-potential discharge electrode and a grounded counter electrode. The discharge electrode and the counter electrode are disposed in parallel to each other and inclined to an opening surface.

The dust collector described in Patent Literature 2 includes an ionizer that charges particles in the air. The ionizer includes a discharge electrode and a counter electrode. The counter electrode is obliquely disposed with respect to an opening surface. The discharge electrode is formed of an ionizing line.

JP 2005 074389 A discloses a two step electric dust collector for an air conditioner comprising an ionizer. The counter electrodes of the ionizer are installed so as to be tilted in a depth direction of the electric dust collector.

### Citation List

### Patent Literature

Patent Literature 1: Japanese Patent No. 3,605,206
Patent Literature 2: Japanese Patent No. 4,252,405

### Summary of Invention

### Technical Problem

In the dust collector described in Patent Literature 2, the discharge electrode, which is formed of an ionizing line, disadvantageously tends to be broken. If a broken discharge electrode comes into contact with the counter electrode, spark discharge may occur or the apparatus may stop operating. Further, since an ionizing line is used as the discharge electrode, increasing the inclination of the counter electrode to reduce the thickness of the apparatus disadvantageously causes an increase in frequency of occurrence of spark discharge between the discharge electrode and the counter electrode.

In the dust collector described in Patent Literature 1, since the front end of the discharge electrode has a round shape, it is difficult to reduce the thickness of the apparatus because the round portion remains. Further, the dust collector described in Patent Literature 1 has the same problem as Patent Literature 2, that is, increasing the inclination of the counter electrode to reduce the thickness of the apparatus disadvantageously increases occurrence of spark discharge.

The present invention was made to solve the problems described above, and an object of the present invention is to provide an electric field and discharge generator capable of forming a stable, uniform electric field with the thickness of the apparatus reduced and greatly reducing the occurrence of spark discharge or any other abnormal discharge produced between a discharge electrode and a counter electrode.

### Solution to Problem

An electric field and discharge generator of the invention is a generator which comprises a first frame having a first opening for introducing outside air inside, a second frame having a second opening for exhausting inside air outside, a discharge electrode having a plate-like shape and so disposed that the discharge electrode is perpendicular to an opening surface of the first frame and an opening surface of the second frame, and a counter electrode having an electrode portion that has a plate-like shape, the electrode portion obliquely disposed with respect to the opening surface of the first frame and the opening surface of the second frame.

### Advantageous Effects of Invention

According to the present invention, a stable, uniform electric field can be formed with the thickness of the apparatus reduced, and the occurrence of spark discharge or any other abnormal discharge produced between the discharge electrode and the counter electrode can be greatly reduced.

### Brief Description of Drawings

Figure 1 is an exploded perspective view showing an electric field and discharge generator in a first embodiment according to the present invention.
Figure 2 is a cross-sectional view of the electric field and discharge generator in the first embodiment according to the present invention.
Figure 3 is a perspective view showing an electrode support of the electric field and discharge generator in the first embodiment according to the present invention.
Figure 4 is a perspective view showing a main portion of the electric field and discharge generator in the first embodiment according to the present invention.
Figure 5 is a perspective view showing the counter electrode of the electric field and discharge generator in the first embodiment according to the present invention.
Figure 6 is a diagram to explain a structure of supporting an electrode portion of the counter electrode.
Figure 7 is a diagram showing another structure of supporting the electrode portion of the counter electrode.
Figure 8 is a diagram showing another structure of supporting the electrode portion of the counter electrode.
Figure 9 is a diagram to explain the positional relationship between a discharge electrode and the counter electrode.
Figure 10 is a diagram showing the positional relationship between the discharge electrode and the counter electrode in the first embodiment according to the present invention.
Figure 11 is a diagram showing a different positional relationship between the discharge electrode and the counter electrode.
Figure 12 is a cross-sectional view of an air conditioner that accommodates the electric field and discharge generator having the configuration shown in Figure 1.

### Description of Embodiments

The present invention will be described in detail with reference to the accompanying drawings. In the drawings, the same or corresponding portions have the same reference signs. Redundant descriptions are appropriately simplified or omitted.

### First Embodiment

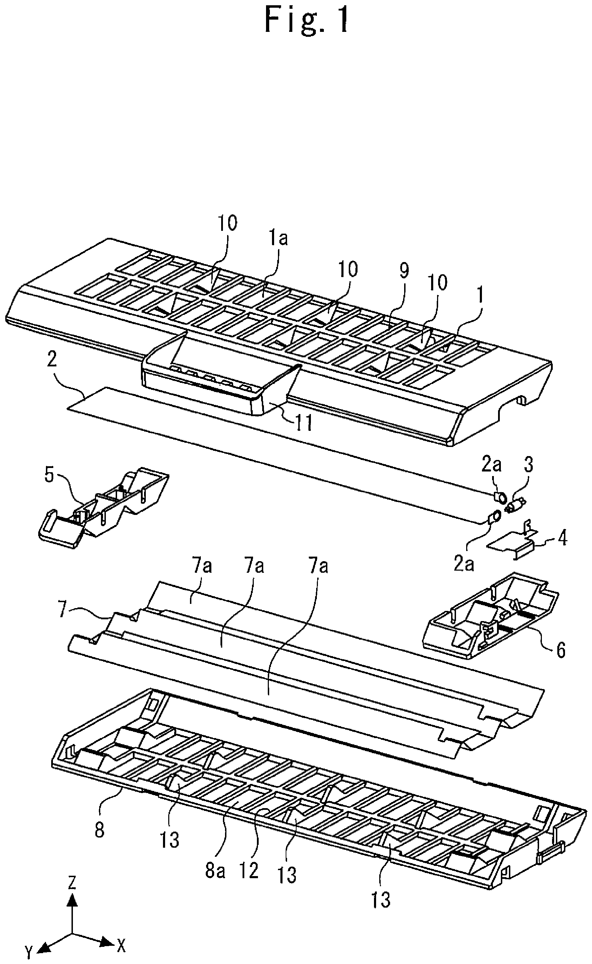

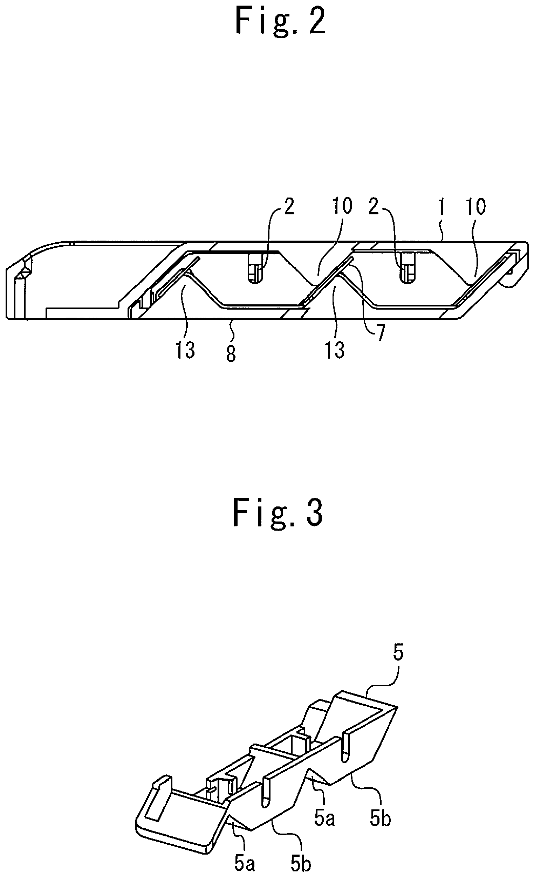

Figure 1 is an exploded perspective view showing an electric field and discharge generator in a first embodiment according to the present invention. Figure 2 is a cross-sectional view of the electric field and discharge generator in the first embodiment according to the present invention. In Figure 1, the upper side represents the upstream side of the apparatus, and the lower side represents the downstream side of the apparatus.

The electric field and discharge generator includes an upper frame 1, a discharge electrode 2, a spring 3, a power feeder 4, electrode supports 5 and 6, a counter electrode 7, and a lower frame 8, as shown in Figures 1 and 2. The exterior shape of the electric field and discharge generator is formed by the upper frame 1 and the lower frame 8.

The upper frame 1 forms a portion disposed on the most upstream side of the apparatus. The entire upper frame 1 is made, for example, of a resin. The upper frame 1 has openings 1a for introducing outside air into the apparatus. The upper frame 1 further has a lattice 9, upper ribs 10, and a handle 11.

The lattice 9 is provided to prevent a finger of a person from accessing the interior of the apparatus through any of the openings 1a. Holes produced by the lattice 9 form the openings 1a. The openings 1a are formed through the upper surface (surface parallel to X-Y plane in Figure 1) of the upper frame 1 across a region having a predetermined width (distance in X-axis direction in Figure 1) and a predetermined depth (distance in Y-axis direction in Figure 1).

Each of the upper ribs 10 is formed, for example, of a plate-shaped member having a triangular shape. The upper ribs 10 are disposed in the lattice 9, and each of them has a front end protruding inward (downward) in the apparatus. The upper ribs 10 are so disposed that they are perpendicular to the surface of the upper frame 1 where the openings 1a are formed (hereinafter also referred to as an "opening surface" of the upper frame 1). The entire lattice 9 and upper ribs 10 are made, for example, of a resin and formed integrally with a main body of the upper frame 1.

The handle 11 is provided to allow a person to grasp the apparatus with a hand.

The lower frame 8 forms a portion disposed on the most downstream side of the apparatus. The entire lower frame 8 is made, for example, of a resin. The lower frame 8 has openings 8a for exhausting the air introduced into the apparatus through the openings 1a out of the apparatus. The lower frame 8 has a lattice 12 and lower ribs 13.

The lattice 12 is provided to prevent a finger of a person from accessing the interior of the apparatus through any of the openings 8a. Holes produced by the lattice 12 form the openings 8a. The openings 8a are formed through the lower surface (surface parallel to X-Y plane in Figure 1) of the lower frame 8 across a region having a predetermined width (distance in X-axis direction in Figure 1) and a predetermined depth (distance in Y-axis direction in Figure 1). The surface of the lower frame 8 where the openings 8a are formed (opening surface) is disposed in parallel to the opening surface of the upper frame 1. When no part is present in the apparatus, the openings 8a are set apart from the openings 1a in the height direction (Z-axis direction in Figure 1) by a predetermined distance and face the openings 1a.

Each of the lower rib 13 is formed, for example, of a plate-shaped member having a triangular shape. Each of the lower ribs 13 and the corresponding upper rib 10 form a pair of support members. The lower ribs 13 are disposed in the lattice 12, and each of them has a front end protruding inward (upward) in the apparatus. The lower ribs 13 are so disposed that they are perpendicular to the opening surface of the lower frame 8. The entire lattice 12 and lower ribs 13 are made, for example, of a resin and formed integrally with a main body of the lower frame 8.

A main portion of the discharge electrode 2 is formed of an elongated plate-shaped member made of a metal. The metal of which the discharge electrode 2 is made is preferably, for example, tungsten, copper, nickel, stainless steel, zinc, iron, or molybdenum. The discharge electrode 2 may be made of an alloy primarily containing any of the metals described above or any of the metals described above the surface of which is plated with silver, gold, platinum, or any other precious metal.

The discharge electrode 2 has a rectangular cross-sectional shape surrounded by shorter sides and longer sides. Each of the shorter sides of the cross section of the discharge electrode 2 has a length ranging, for example, from 0.01 to 0.1 mm, and each of the longer sides of the cross section of the discharge electrode 2 has a length ranging, for example, from 0.1 to 1.0 mm. A ring-shaped terminal 2a is attached to each end of the discharge electrode 2. The discharge electrode 2 is folded back in intermediate positions about two to four times and accommodated in the apparatus.

The spring 3 is provided to exert a predetermined tensile force on the discharge electrode 2. The spring 3 is formed of a member made of a metal. Each of the ends of the discharge electrode 2 is connected to the spring 3 via the corresponding terminal 2a. The discharge electrode 2, which is pulled in the longitudinal direction thereof by the spring 3, is attached to the power feeder 4.

The power feeder 4 is formed of a member made of a metal. The power feeder 4 is connected to a power supply (not shown) for supplying a voltage ranging from 4 to 7 kV to the discharge electrode 2 (between discharge electrode 2 and counter electrode 7).

Figure 3 is a perspective view showing the electrode support (electrode support 5) of the electric field and discharge generator in the first embodiment according to the present invention.

The electrode supports 5 and 6 are provided to support the discharge electrode 2 in the apparatus. The entire electrode supports 5 and 6 are made, for example, of a resin. The electrode support 5 is attached to an end portion of the lower frame 8 on one side thereof. The electrode support 6 is attached to an end portion of the lower frame 8 on the other side thereof. The discharge electrode 2 is supported by the electrode support 5 on the one side of the apparatus and supported by the electrode support 6 on the other side of the apparatus. Each of the electrode supports 5 and 6 is provided with a member for folding back the discharge electrode 2 and a member for placing the discharge electrode 2 in an appropriate position.

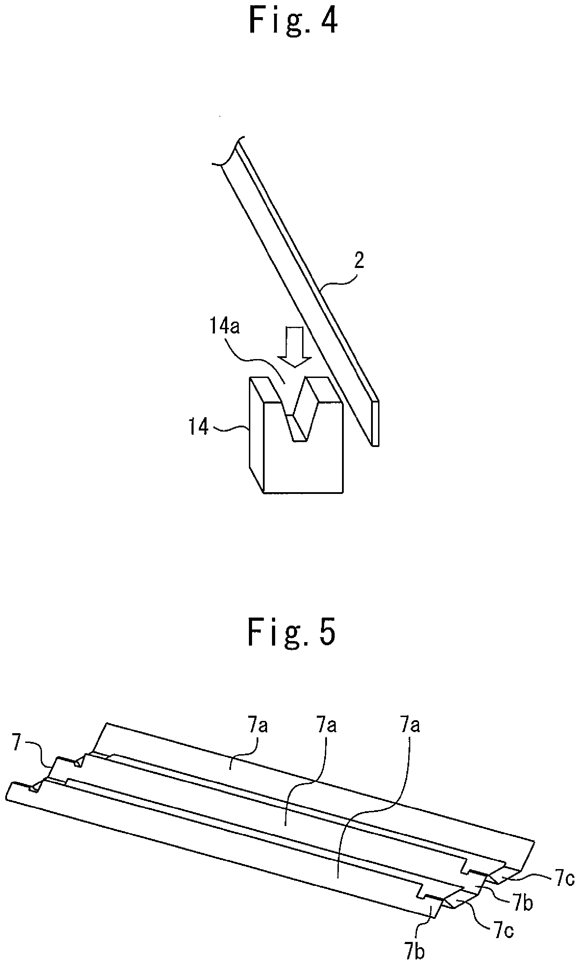

Figure 4 is a perspective view showing a main portion of the electric field and discharge generator in the first embodiment according to the present invention. Figure 4 shows a member that supports the discharge electrode 2 in a central portion of the apparatus.

The discharge electrode 2 is supported by the electrode support 5 on the one end side of the apparatus and supported by the electrode support 6 on the other end side of the apparatus, as described above. The discharge electrode 2 is supported by support ribs 14 in a central portion of the apparatus. A slit 14a (recess) open upward is formed in each of the support ribs 14. The opening of the slit 14a has a width ranging from 0.1 to 1.0 mm. The discharge electrode 2 in the upright position is inserted into the slits 14a and supported by the support rib 14 in the upright position.

The support ribs 14 are provided in any one or ones of the upper frame 1, the lower frame 8, the electrode support 5, and the electrode support 6. The discharge electrode 2, when it is supported by the electrode supports 5 and 6 in the end portions of the apparatus and supported by the support ribs 14 in the central portion of the apparatus, is disposed as a whole perpendicular to the opening surface of the upper frame 1 (and the opening surface of the lower frame 8). When the discharge electrode 2 is appropriately supported in the apparatus, the surface that forms one of the shorter sides of the cross section (end surface) of the discharge electrode 2 faces the openings 1a, and the surface that forms the other shorter side (end surface) of the discharge electrode 2 faces the openings 8a.

Figure 5 is a perspective view showing the counter electrode of the electric field and discharge generator in the first embodiment according to the present invention.

The counter electrode 7 is produced by cutting and bending a plate-shaped member made of a metal. The metal of which the counter electrode 7 is made is preferably, for example, tungsten, copper, nickel, stainless steel, zinc, iron, or molybdenum. The counter electrode 7 may be made of an alloy primarily containing any of the metals described above or any of the metals described above the surface of which is plated with silver, gold, platinum, or any other precious metal.

The counter electrode 7 includes, for example, electrode portions 7a, bent portions 7b, and horizontal portions 7c.

Each of the electrode portions 7a has a plate-like shape having a width substantially equal to the width of each of the openings 1a (distance in X-axis direction in Figure 1) or greater than the width of each of the openings 1a. A plurality of the electrode portions 7a are provided in a row in the depth direction of the apparatus (Y-axis direction in Figure 1). The electrode portions 7a are arranged at equal intervals in parallel to each other. The electrode portions 7a are so obliquely disposed that they are inclined to the opening surface of the upper frame 1 (and the opening surface of the lower frame 8) by a predetermined angle. In the present embodiment, the electrode portions 7a are so disposed that they are inclined to the opening surface of the upper frame 1 by an angle ranging from 45 to 60 degrees.

Producing the counter electrode 7 by using a metal plate allows more precise formation of the electrode portions 7a than in a case where the counter electrode 7 is produced in a resin molding process. Further, a counter electrode 7 formed of a metal plate greatly suppresses a secular change in the inclination of the electrode portions 7a.

The ends of adjacent electrode portions 7a are connected to each other via the bent portions 7b and horizontal portion 7c. Each of the bent portions 7b is so bent that it protrudes toward the upper frame 1 to form a triangular shape. The bent portion 7b is provided at each end of each of the electrode portions 7a. Each of the horizontal portions 7c has a flat-plate-like shape and is disposed in parallel to the opening surface of the upper frame 1. The horizontal portion 7c connects adjacent bent portions 7b to each other. Each end of the counter electrode 7 is formed of the bent portions 7b and the horizontal portions 7c alternately arranged to form a wavy shape.

The counter electrode 7 is fixed in a predetermined position when an end portion thereof on one side (that is, the wavy portion formed of the bent portions 7b and the horizontal portions 7c) is sandwiched between the electrode support 5 and the lower frame 8 from above and below. Triangular recess portions 5a corresponding to the bent portions 7b and flat portions 5b corresponding to the horizontal portions 7c are alternately formed along the lower surface of the electrode support 5. Similarly, the counter electrode 7 is fixed in a predetermined position when an end portion thereof on the other side is sandwiched between the electrode support 6 and the lower frame 8 from above and below. Triangular recess portions corresponding to the bent portions 7b and flat portions corresponding to the horizontal portions 7c are alternately formed along the lower surface of the electrode support 6.

When the counter electrode 7 is appropriately disposed in the apparatus, the side surfaces of each of the electrode portions 7a obliquely face the openings 1a and 8a. The bent portions 7b and the horizontal portions 7c do not face the openings 1a and 8a. The bent portions 7b and the horizontal portions 7c are not exposed to a portion where air flows in the apparatus. The bent portions 7b and the horizontal portions 7c therefore do not block an air flow generated in the apparatus.

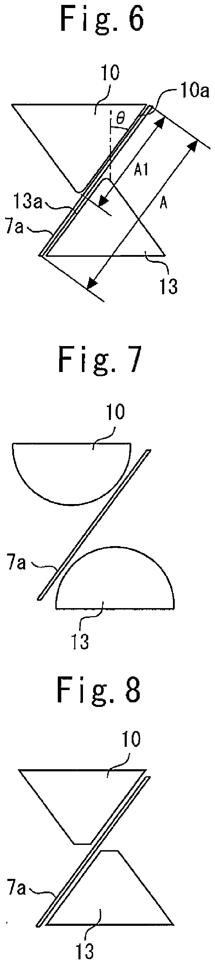

A structure of supporting the electrode portions 7a will next be described in detail with also reference to Figures 6 to 8.

Figure 6 is a diagram to explain a structure of supporting the electrode portion of the counter electrode. An intermediate portion of each of the electrode portions 7a of the counter electrode 7 is supported by the upper ribs 10 and lower ribs 13.

An end surface 10a of each of the upper ribs 10 is so formed that it is inclined to the opening surface of the upper frame 1, as shown in Figure 6. The upper rib 10 is so disposed that the end surface 10a faces the upward-facing side surface of the electrode portion 7a (side surface that faces openings 1a) with a slight gap. That is, the end surfaces 10a of the upper ribs 10 are formed in accordance with the inclination of the electrode portions 7a of the counter electrode 7 appropriately disposed in the apparatus.

The upper ribs 10 and the lower ribs 13 form a pair of support members. The lower ribs 13 are disposed in parallel to the upper ribs 10 so that each of the lower ribs 13 and the corresponding upper rib 10 are present in the same plane. Each of the lower ribs 13 is so formed that an end surface 13a thereof is in parallel to the corresponding end surface 10a. The end surface 13a is therefore obliquely disposed with respect to the opening surface of the upper frame 1. The lower rib 13 is so disposed that the end surface 13a faces the downward-facing side surface of the electrode portion 7a (side surface that faces openings 8a) with a slight gap. That is, the end surfaces 13a of the lower ribs 13 are formed in accordance with the inclination of the electrode portions 7a of the counter electrode 7 appropriately disposed in the apparatus.

Each of the electrode portions 7a is so supported that the intermediate portion thereof is sandwiched by the upper ribs 10 and lower ribs 13, whereby the inclination of the electrode portion 7a is maintained. Each of the upper ribs 10 and the lower ribs 13 may have any shape having an end surface that holds the electrode portion 7a. Figures 7 and 8 are diagrams showing other structures of supporting the electrode portion of the counter electrode. Figure 7 shows a case where each of the upper ribs 10 and the lower ribs 13 has a semispherical shape. Figure 8 shows a case where each of the upper ribs 10 and the lower ribs 13 has a trapezoidal shape.

Each of the upper ribs 10 is preferably so configured that the length of the end surface 10a (A1 in Figure 6) is at least one-third the inclined length (that is, the distance between the edge portion of the end surface 10a that is close to the opening surface of the upper frame 1 and the edge portion of the end surface 10a that is close to the opening surface of the lower frame 8 (A in Figure 6)) of the electrode portion 7a. Similarly, each of the lower ribs 13 is also preferably so configured that the length of the end surface 13a is at least one-third the inclined length of the electrode portion 7a. The configuration described above allows the electrode portion 7a to be received by a wide area of each of the ribs and hence to be held in a reliable manner. Further, when the length of each of the end surfaces 10a and 13a is long enough, a base portion of each of the ribs can be thick and strong, which prevents the ribs from being broken, whereby the electrode portion 7a can be held in an appropriate position over a long period.

In the present embodiment, the lower end of each of the upper ribs 10 is positioned below the upper end of the corresponding lower rib 13 by about 1 to 10 mm. That is, a lower portion of the upper rib 10 and an upper portion of the lower rib 13 overlap with each other in the height direction (Z-axis direction in Figure 1). The configuration described above allows the electrode portion 7a to be securely held from above and below, whereby the electrode portion 7a can be held with a greater force.

Further, the electrode portions 7a are preferably held at an inclination angle that does not cause the air flow in the apparatus (flow of air introduced through the openings 1a into the apparatus and exhausted through the openings 8a out of the apparatus) to be blocked. The inclination of the electrode portions 7a is preferably so set that the inclination angle with respect to the direction in which the air flows into the apparatus (direction of normal to opening surface of upper frame 1) (θ in Figure 6) is 45 degrees or smaller. When the apparatus is incorporated in a product, the apparatus blocks an air flow in the product, and the power consumption of the product therefore increases. When the electrode portions 7a are held within the inclination angle range described above, the apparatus incorporated in a product only increases the power consumption of the product by 1W or lower.

In the present embodiment, the angle between the electrode portions 7a and the opening surface of the upper frame 1 (90-θ) is set at a value ranging from 45 to 60 degrees, as described above. The angle of the electrode portions 7a with respect to the direction in which the air flows into the apparatus is therefore 45 degrees or smaller.

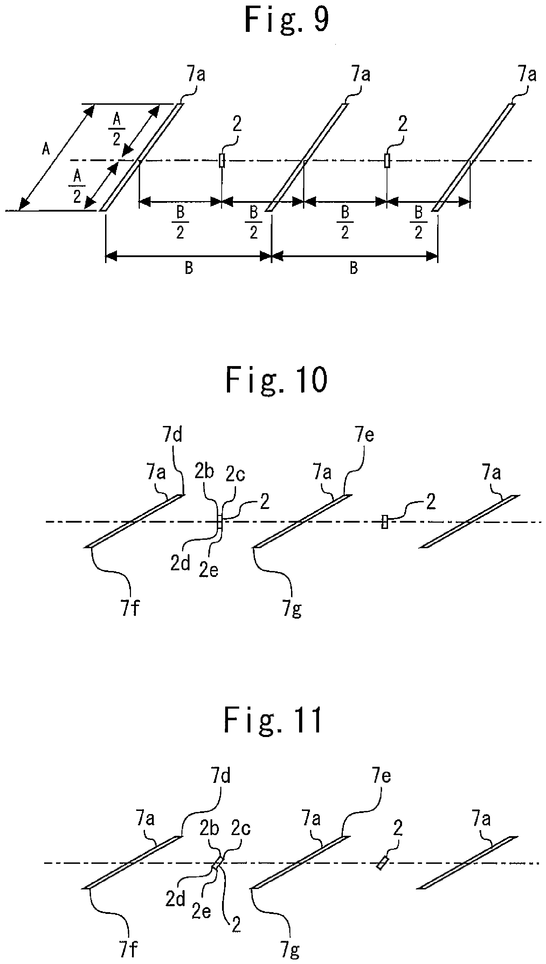

The positional relationship between the discharge electrode 2 and the counter electrode 7 will next be described in detail with also reference to Figure 9. Figure 9 is a diagram to explain the positional relationship between the discharge electrode and the counter electrode.

The electrode portions 7a of the counter electrode 7 are arranged at equal intervals in the depth direction of the apparatus (Y-axis direction in Figure 1). Further, the electrode portions 7a are obliquely disposed with respect to the opening surface of the upper frame 1 and the opening surface of the lower frame 8. The discharge electrode 2 is so disposed that it is perpendicular to the opening surface of the upper frame 1 and the opening surface of the lower frame 8. The end surface of the discharge electrode 2 on one side faces the openings 1a, and the end surface of the discharge electrode 2 on the other side faces the openings 8a. The side surfaces of the discharge electrode 2 are so disposed that they are perpendicular to the opening surface of the upper frame 1.

Reference sign A in Figure 9 represents the inclined length of each of the electrode portions 7a, as described above. Reference sign B in Figure 9 represents the intervals at which the electrode portions 7a are arranged, that is, the distance between the electrode portions 7a in the depth direction (direction parallel to opening surface of upper frame 1).

The discharge electrode 2 is disposed between two electrode portions 7a parallel to each other. One side surface of the discharge electrode 2 therefore obliquely faces the side surface of one of the two electrode portions 7a described above. The other side surface of the discharge electrode 2 obliquely faces the side surface of the other one of the two electrode portions 7a.

The discharge electrode 2 is disposed in the exactly middle position between the two electrode portions 7a parallel to each other. Specifically, the discharge electrode 2 is disposed at the level of a straight line that extends at the middle of the height of the two adjacent electrode portions 7a (height A/2). The discharge electrode 2 is so disposed that the distances to the two electrode portions 7a are equal to each other. That is, the discharge electrode 2 is so disposed that the horizontal distance to one of the electrode portions 7a and the horizontal distance to the other electrode portion 7a are both B/2.

Further, the counter electrode 7 is so configured that the inclined length A of each of the electrode portions 7a is at least one-half the intervals B at which the electrode portions 7a are arranged (A≥B/2).

In the thus configured electric field and discharge generator, when a blowing fan (not shown) provided external to the apparatus is driven, outside air is introduced through the openings 1a into the apparatus. In this process, airborne germs, fungi, viruses, and the like are also introduced through the openings 1a into the apparatus. In the electric field and discharge generator, the counter electrode 7 is grounded and a voltage ranging from 4 to 7 kV is applied to the discharge electrode 2. An electric field is formed between the discharge electrode 2 and the counter electrode 7, and corona discharge occurs.

The germs, fungi, and viruses having entered the apparatus through the openings 1a are applied to the electric field and discharge and are destroyed and dead when they pass through the space between the discharge electrode 2 and the counter electrode 7. The dead germs, fungi, and viruses are exhausted with air through the openings 8a out of the apparatus. The in-room, airborne, live germs, fungi, and viruses can thus be removed or deactivated.

The function of the electric field and discharge generator having the configuration described above will next be specifically described with also reference to Figures 10 and 11.

Figure 10 is a diagram showing the positional relationship between the discharge electrode and the counter electrode in the first embodiment according to the present invention. The positional relationship between the discharge electrode 2 and the counter electrode 7 shown in Figure 10 is the same as the positional relationship in the state shown in Figure 9. Figure 11 is a diagram showing a different positional relationship between the discharge electrode and the counter electrode. Figure 11 shows an arrangement in which the discharge electrode 2 is so inclined from the state shown in Figure 10 that the discharge electrode 2 and the counter electrode 7 are nearly parallel to each other.

In Figures 10 and 11, reference signs 2b to 2e represent angled portions formed by the side surfaces and the end surfaces of the discharge electrode 2. Reference signs 7d to 7g represent angled portions formed by the side surfaces and the end surfaces of each of the electrode portions 7a.

When a voltage ranging from 4 to 7 kV is applied between the discharge electrode 2 and the counter electrode 7, discharge occurs between the angled portions of the discharge electrode 2 and the electrode portions 7a. Since the electrode portions 7a of the counter electrode 7 are inclined, the distance between the discharge electrode 2 and the counter electrode 7 is shorter in some places than in others. In the positions where the distance is shorter, the intensity of the electric field is greater than that in the other positions, whereby occurrence of discharge is more likely.

In the arrangement shown in Figure 10, the angled portion 2b of the discharge electrode 2 is located in a position close to the angled portion 7d of the electrode portion 7a. Discharge therefore tends to occur between the angled portions 2b and 7d.

In the arrangement shown in Figure 11 as well, the angled portion 2b of the discharge electrode 2 is located in a position close to the angled portion 7d of the electrode portion 7a. In the arrangement shown in Figure 11, however, the angled portion 2d of the discharge electrode 2 is also located in a position close to the angled portion 7d of the electrode portion 7a. Discharge therefore occurs in the arrangement shown in Figure 11 between the angled portions 2b and 7d and between the angled portions 2d and 7d, resulting in discharge interference. When discharge interference occurs, spark discharge tends to occur. Further, when discharge interference occurs, the amount of produced ozone increases, and the magnitude of discharge sound increases.

In the arrangement shown in Figure 10, in which only one angled portion of the discharge electrode 2 is located in a position close to one electrode portion 7a, no discharge interference occurs. Employing the arrangement shown in Figure 10 produces a stable discharge state between the discharge electrode 2 and the counter electrode 7. That is, the following disadvantageous events can be suppressed: occurrence of spark discharge; an increase in the amount of produced ozone; and an increase in the magnitude of discharge sound.

The same holds true for a lower end portion of the discharge electrode 2.

In the arrangement shown in Figure 10, the angled portion 2e of the discharge electrode 2 is located in a position close to the angled portion 7g of the electrode portion 7a. The angled portion 2e is a diagonally opposite angled portion to the angled portion 2b, which is located in the position closest to the one of the electrode portions 7a adjacent to the discharge electrode 2. When the discharge electrode 2 and the counter electrode 7 are so disposed that they satisfy the positional relationship shown in Figure 9, the distance between the angled portion 2b and one of the adjacent electrode portions 7a is equal to the distance between the angled portion 2e and the other adjacent electrode portion 7a. Discharge therefore tends to occur between the angled portions 2e and 7g as well.

In the arrangement shown in Figure 11, the angled portion 2e of the discharge electrode 2 is located in a position close to the angled portion 7g of the electrode portion 7a as well. In the arrangement shown in Figure 11, however, the angled portion 2c of the discharge electrode 2 is also located in a position close to the angled portion 7g of the electrode portion 7a. Discharge therefore occurs in the arrangement shown in Figure 11 between the angled portions 2e and 7g and between the angled portions 2c and 7g, resulting in discharge interference. When discharge interference occurs, spark discharge tends to occur. Further, when discharge interference occurs, the amount of produced ozone increases, and the magnitude of discharge sound increases.

Even when the discharge electrode 2 is obliquely disposed with respect to the opening surface of the upper frame 1 (and opening surface of lower frame 8), occurrence of discharge interference can be suppressed by disposing the electrode portions 7a of the counter electrode 7 in such a way that they are nearly perpendicular to the opening surface of the upper frame 1. To reduce the thickness of the apparatus, however, the electrode portions 7a of the counter electrode 7 need to be inclined to the opening surface of the upper frame 1 so that the distance between the openings 1a and the openings 8a is shortened. On the other hand, when the inclination angle of the electrode portions 7a of the counter electrode 7 is too large, the angled portions of the discharge electrode 2 and the angled portions of the electrode portions 7a excessively approach each other. In this case, spark discharge disadvantageously tends to occur.

To reduce the thickness of the apparatus and suppress the occurrence of spark discharge at the same time, an optimum arrangement is as follows: The electrode portions 7a of the counter electrode 7 are inclined to the opening surface of the upper frame 1 (and opening surface of lower frame 8) by an angle ranging from 45 to 60 degrees. When the electrode portions 7a are inclined by an angle ranging from 45 to 60 degrees, if the apparatus is incorporated, for example, in an indoor unit of an air conditioner, the apparatus does not block an air flow in the indoor unit and adversely affect cooling/heating performance of the air conditioner. When the discharge electrode 2 is so disposed that it is perpendicular to the opening surface of the upper frame 1 (and opening surface of lower frame 8), the efficiency at which the apparatus is assembled is also improved.

Further, in this apparatus, the discharge electrode 2 is disposed in the exactly middle position between adjacent electrode portions 7a parallel to each other. As a result, no portion between the discharge electrode 2 and the counter electrode 7 has a particularly high-intensity electric field, but a stable electric field can be formed and a stable discharge state can be maintained.

To suppress the occurrence of spark discharge, an increase in the amount of produced ozone, and an increase in the magnitude of discharge sound, the inclined length A of each of the electrode portions 7a is preferably at least one-half the intervals B at which the electrode portions 7a are arranged. When the inclined length A of each of the electrode portions 7a is shorter than B/2, the angled portions of the discharge electrode 2 and the angled portions of the electrode portions 7a excessively approach each other, a uniform electric field may not be produced. Any portion where the intensity of the electric field is particularly high contributes to the occurrence of spark discharge, an increase in the amount of produced ozone, and an increase in the magnitude of discharge sound.

As described above, according to the electric field and discharge generator having the configuration described above, a stable, uniform electric field can be formed with the thickness of the apparatus reduced, whereby occurrence of spark discharge or any other abnormal discharge produced between the discharge electrode 2 and the counter electrode 7 can be greatly suppressed and a stable discharge state can be maintained for a long period.

### Second Embodiment

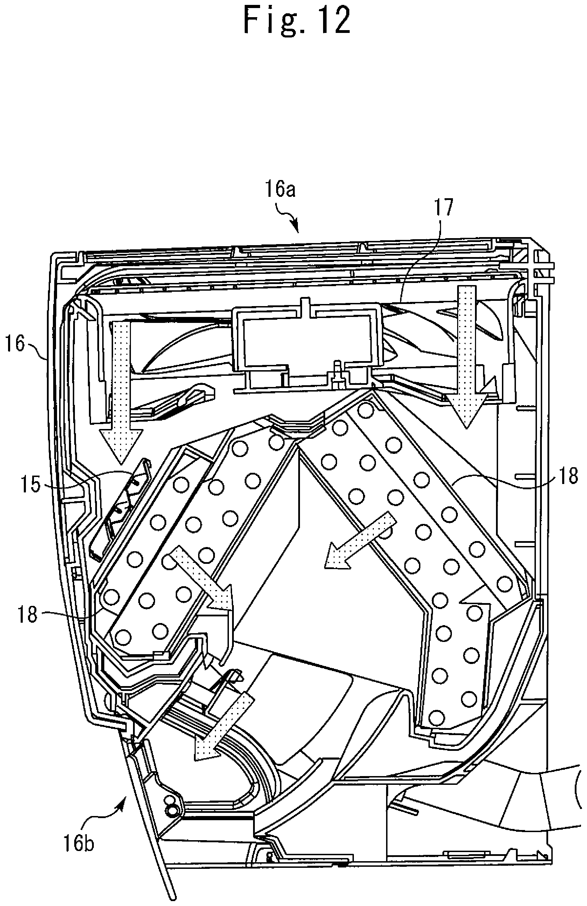

The present embodiment will be described with reference to a case where the electric field and discharge generator having the configuration described above is incorporated in an indoor unit of an air conditioner. Figure 12 is a cross-sectional view of an air conditioner that accommodates the electric field and discharge generator having the configuration shown in Figure 1.

In Figure 12, reference sign 15 denotes the electric field and discharge generator described in the first embodiment. Reference sign 16 denotes a main body of the indoor unit of the air conditioner. An air inlet 16a is formed through the upper surface of the main body 16. An air outlet 16b is formed through a lower front surface of the main body 16. The main body 16 accommodates a blower fan 17, a heat exchanger 18, and the electric field and discharge generator 15.

The blower fan 17 produces an air flow in the main body 16. The blower fan 17 is formed, for example, of a propeller fan. The blower fan 17 is disposed below the air inlet 16a in the vicinity thereof.

The heat exchanger 18 is disposed below the blower fan 17 but above the air outlet 16b. The heat exchanger 18 is so obliquely disposed in the main body 16 that the cross section of the heat exchanger 18 has an inverted V-like shape.

The electric field and discharge generator 15 is disposed below the blower fan 17 but above the heat exchanger 18. The electric field and discharge generator 15 is obliquely disposed along the inclination of the upper surface of the heat exchanger 18. In the thus disposed electric field and discharge generator 15, the opening surface of the upper frame 1 and the opening surface of the lower frame 8 are inclined and the electrode portions 7a of the counter electrode 7 are therefore so fixed that they are substantially vertical.

In the air conditioner having the configuration described above, when the blower fan 17 operates, outside air is introduced through the air inlet 16a into the main body 16. In Figure 12, an air flow produced when the blower fan 17 operates is indicated by thick arrows. The air introduced into the main body 16 is delivered downward by the blower fan 17 and flows into the electric field and discharge generator 15 or the heat exchanger 18.

As described above, the electric field and discharge generator 15 is obliquely disposed along the upper surface of the heat exchanger 18. As a result, the air delivered by the blower fan 17 flows into the electric field and discharge generator 15 in an oblique direction and travels through the interior of the apparatus along the inclination of the electrode portions 7a. The air exhausted through the openings 8a of the electric field and discharge generator 15 flows into the heat exchanger 18. The air having passed through the heat exchanger 18 is exhausted through the air outlet 16b out of the main body 16.

According to the air conditioner having the configuration described above, the electric field and discharge generator 15 disposed in the interior of the main body 16 can remove and deactivate in-room, airborne germs, fungi, and viruses. Since the air delivered by the blower fan 17 flows into the electric field and discharge generator 15 in an oblique direction, the electric field and discharge generator 15 does not produce a large amount of pressure loss. Therefore, the electric field and discharge generator 15 disposed in the interior of an air conditioner will not greatly decrease the cooling/heating performance of the air conditioner.

### Industrial Applicability

Any of the electric field and discharge generators according to the present invention can be incorporated in a room air conditioner, a package air conditioner, a cleaner, a hand dryer, an air cleaner, a humidifier, a dehumidifier, a refrigerator, and other similar products.

### Reference Signs List

1 upper frame
1a, 8a opening
2 discharge electrode
2a terminal
2b, 2c, 2d, 2e angled portion
3 spring
4 power feeder
5, 6 electrode support
5a recess portion
5b flat portion
7 counter electrode
7a electrode portion
7b bent portion
7c horizontal portion
7d, 7e, 7f, 7g angled portion
8 lower frame
9, 12 lattice
10 upper rib
10a, 13a end surface
11 handle
13 lower rib
14 support rib
14a slit
15 electric field and discharge generator
16 main body
16a air inlet
16b air outlet
17 blower fan
18 heat exchanger

## Claims

1. An electric field and discharge generator comprising:
a first frame (1) having a first opening (1a) for introducing air;
a second frame (8) having a second opening (8a) for exhausting air;
a discharge electrode (2) having a plate-like shape and so disposed that the discharge electrode (2) is perpendicular to an opening surface of the first frame (1) and an opening surface of the second frame (8), the discharge electrode (2) having a rectangular cross-sectional shape surrounded by shorter sides and longer sides; and
a counter electrode (7) having a first electrode portion (7a) and a second electrode portion, the first electrode portion (7a) and the second electrode portion having a plate-like shape and disposed in parallel to each other, the first electrode portion (7a) and the second electrode portion obliquely disposed with respect to the opening surface of the first frame (1) and the opening surface of the second frame (8),
the discharge electrode (2) having one side surface obliquely facing the first electrode portion (7a) and the other side surface obliquely facing the second electrode portion.

2. The electric field and discharge generator according to claim 1,
wherein the discharge electrode (2) is so disposed in the middle position between the first electrode portion (7a) and the second electrode portion that the distance between the first electrode portion (7a) and a first angled portion (2b) of the discharge electrode (2) that is closest to the first electrode portion (7a) is equal to the distance between the second electrode portion and a second angled portion (2e) of the discharge electrode (2) that is diagonally opposite to the first angled portion (2b).

3. The electric field and discharge generator according to claim 1 or 2,
wherein the distance between one edge of the first electrode portion (7a) that is located in a position close to the opening surface of the first frame (1) and the other edge of the first electrode portion (7a) that is located in a position close to the opening surface of the second frame (8) is a value greater than or equal to B/2, where B is the distance between the first electrode portion (7a) and the second electrode portion in a direction parallel to the opening surface of the first frame (1).

4. The electric field and discharge generator according to any of claims 1 to 3,
wherein the first electrode portion (7a) and the second electrode portion are obliquely disposed with respect to the opening surface of the first frame (1) and the opening surface of the second frame (8) by an angle ranging from 45 to 60 degrees.

5. An air conditioning apparatus comprising:
the electric field and discharge generator according to any of claims 1 to 4.

6. The air conditioning apparatus according to claim 5, further comprising
a blower fan,
wherein air is introduced into the electric field and discharge generator when the blower fan is driven.

## Patentansprüche

1. Elektrisches-Feld- und Entladungsgenerator, umfassend:
einen ersten Rahmen (1), aufweisend eine erste Öffnung (1a) zum Einleiten von Luft;
einen zweiten Rahmen (8), aufweisend eine zweite Öffnung (8a) zum Abführen von Luft;
eine Entladungselektrode (2), aufweisend eine plattenähnliche Form und so angeordnet, dass die Entladungselektrode (2) senkrecht zu einer Öffnungsoberfläche des ersten Rahmens (1) und einer Öffnungsoberfläche des zweiten Rahmens (8) ist, wobei die Entladungselektrode (2) eine rechteckige Querschnittsform aufweist, die von kürzeren Seiten und längeren Seiten umgeben ist; und
eine Gegenelektrode (7), aufweisend einen ersten Elektrodenabschnitt (7a) und einen zweiten Elektrodenabschnitt, wobei der erste Elektrodenabschnitt (7a) und der zweite Elektrodenabschnitt eine plattenähnliche Form aufweisen und parallel zueinander angeordnet sind, wobei der erste Elektrodenabschnitt (7a) und der zweite Elektrodenabschnitt schräg in Bezug auf die Öffnungsoberfläche des ersten Rahmens (1) und die Öffnungsoberfläche des zweiten Rahmens (8) angeordnet sind,
wobei die Entladungselektrode (2) eine Seitenoberfläche aufweist, die dem ersten Elektrodenabschnitt (7a) schräg zugewandt ist, und die andere Seitenoberfläche dem zweiten Elektrodenabschnitt schräg zugewandt ist.

2. Elektrisches-Feld- und Entladungsgenerator nach Anspruch 1,
wobei die Entladungselektrode (2) in der Mittelposition zwischen dem ersten Elektrodenabschnitt (7a) und dem zweiten Elektrodenabschnitt so angeordnet ist, dass der Abstand zwischen dem ersten Elektrodenabschnitt (7a) und einem ersten gewinkelten Abschnitt (2b) der Entladungselektrode (2), der zum ersten Elektrodenabschnitt (7a) am nächsten ist, gleich dem Abstand zwischen dem zweiten Elektrodenabschnitt und einem zweiten gewinkelten Abschnitt (2e) der Entladungselektrode (2) ist, der zum ersten gewinkelten Abschnitt (2b) diagonal gegenüberliegend ist.

3. Elektrisches-Feld- und Entladungsgenerator nach Anspruch 1 oder 2, wobei der Abstand zwischen einer Kante des ersten Elektrodenabschnitts (7a), der sich an einer Position nahe der Öffnungsoberfläche des ersten Rahmens (1) befindet, und der anderen Kante des ersten Elektrodenabschnitts (7a), der sich an einer Position nahe der Öffnungsoberfläche des zweiten Rahmens (8) befindet, ein Wert ist größer als oder gleich wie B/2, wobei B der Abstand zwischen dem ersten Elektrodenabschnitt (7a) und dem zweiten Elektrodenabschnitt in einer Richtung parallel zur Öffnungsoberfläche des ersten Rahmens (1) ist.

4. Elektrisches-Feld- und Entladungsgenerator nach einem der Ansprüche 1 bis 3,
wobei der erste Elektrodenabschnitt (7a) und der zweite Elektrodenabschnitt in Bezug auf die Öffnungsoberfläche des ersten Rahmens (1) und die Öffnungsoberfläche des zweiten Rahmens (8) unter einem Winkel im Bereich von 45 bis 60 Grad schräg angeordnet sind.

5. Klimaanlage, umfassend:
den Elektrisches-Feld- und Entladungsgenerator nach einem der Ansprüche 1 bis 4.

6. Klimaanlage nach Anspruch 5, ferner umfassend:
einen Gebläselüfter,
wobei Luft in den Elektrisches-Feld- und Entladungsgenerator eingeleitet wird, wenn der Gebläselüfter angetrieben wird.

## Revendications

1. Générateur de champ électrique et de décharge comprenant :
un premier cadre (1) comportant une première ouverture (1a) pour introduire de l'air ;
un deuxième cadre (8) comportant une deuxième ouverture (8a) pour évacuer l'air
une électrode de décharge (2) ayant une forme similaire à une plaque et disposée de sorte que l'électrode de décharge (2) soit perpendiculaire à une surface d'ouverture du premier cadre (1) et à une surface d'ouverture du deuxième cadre (8), l'électrode de décharge (2) ayant une forme en coupe rectangulaire entourée par des côtés plus courts et des côtés plus longs ; et
une contre-électrode (7) comportant une première partie d'électrode (7a) et une deuxième partie d'électrode, la première partie d'électrode (7a) et la deuxième partie d'électrode ayant une forme similaire à une plaque et étant disposées parallèlement l'une à l'autre, la première partie d'électrode (7a) et la deuxième partie d'électrode étant disposées obliquement par rapport à la surface d'ouverture du premier cadre (1) et à la surface d'ouverture du deuxième cadre (8),
l'électrode de décharge (2) ayant une surface latérale faisant face obliquement à la première partie d'électrode (7a) et l'autre surface latérale faisant face obliquement à la deuxième partie d'électrode.

2. Générateur de champ électrique et de décharge selon la revendication 1,
dans lequel l'électrode de décharge (2) est disposée à la position centrale entre la première partie d'électrode (7a) et la deuxième partie d'électrode de sorte que la distance entre la première partie d'électrode (7a) et une première partie d'angle (2b) de l'électrode de décharge (2) qui est la plus proche de la première partie d'électrode (7a) soit égale à la distance entre la deuxième partie d'électrode et une deuxième partie d'angle (2e) de l'électrode de décharge (2) qui est à l'opposé en diagonale de la première partie d'angle (2b).

3. Générateur de champ électrique et de décharge selon la revendication 1 ou 2,
dans lequel la distance entre un bord de la première partie d'électrode (7a) qui est situé à une position à proximité de la surface d'ouverture du premier cadre (1) et l'autre bord de la première partie d'électrode (7a) qui est situé à une position à proximité de la surface d'ouverture du deuxième cadre (8) est une valeur supérieure ou égale à B/2, où B est la distance entre la première partie d'électrode (7a) et la deuxième partie d'électrode dans une direction parallèle à la surface d'ouverture du premier cadre (1).

4. Générateur de champ électrique et de décharge selon l'une quelconque des revendications 1 à 3,
dans lequel la première partie d'électrode (7a) et la deuxième partie d'électrode sont disposées en oblique par rapport à la surface d'ouverture du premier cadre (1) et à la surface d'ouverture du deuxième cadre (8) selon un angle allant de 45 à 60 degrés.

5. Appareil de climatisation comprenant :
le générateur de champ électrique et de décharge selon l'une quelconque des revendications 1 à 4.

6. Appareil de climatisation selon la revendication 5, comprenant en outre
un ventilateur souffleur,
dans lequel de l'air est introduit dans le générateur de champ électrique et de décharge lorsque le ventilateur souffleur est commandé.
